# EUROPEAN PATENT SPECIFICATION

(11) **EP 3 986 184 B1**
(45) Date of publication and mention of the grant of the patent: **07.06.2023**
(21) Application number: 20732995.4
(22) Date of filing: 19.06.2020
(51) Int. Cl.: A61M 11/00, A61M 11/04, A61M 15/00, A61M 15/06, A24F 40/00, A24F 40/42

(54) **AEROSOL DELIVERY DEVICE**
AEROSOLABGABEVORRICHTUNG
DISPOSITIF DE DISTRIBUTION D'AÉROSOL

(30) Priority: 21.06.2019 EP 19181679
(43) Date of publication of application: 27.04.2022
(73) Proprietor: IMPERIAL TOBACCO LIMITED, Bristol BS3 2LL (GB)
(72) Inventor: AUSTIN, Andrew, Liverpool Merseyside L24 9HP (GB); SAJTOS, Tamas, Liverpool Merseyside L24 9HP (GB); SHENTON, Ross, Liverpool Merseyside L24 9HP (GB)
(74) Representative: Mewburn Ellis LLP
(86) International application number: PCT/EP2020/067259
(87) International publication number: WO 2020/254671

(56) References cited:
- WO-A1-2017/108268
- CN-U- 206 978 764
- CN-U- 207 011 690
- GB-A- 2 566 774
- US-A1- 2016 345 630
- US-A1- 2016 366 944
- US-A1- 2017 281 883
- US-A1- 2017 367 407
- US-A1- 2018 220 707
- US-A1- 2018 297 047

## Description

### Field of the Invention

The present invention relates to an aerosol delivery device and particularly, although not exclusively, to an aerosol delivery device which aims to reduce leakage.

### Background

One form of an aerosol delivery device is a smoking-substitute device, which is an electronic device that permits the user to simulate the act of smoking by producing an aerosol or vapour that is drawn into the lungs through the mouth and then exhaled. The inhaled aerosol or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heating device to produce an aerosol/vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating element. In use, electrical energy is supplied from the power source to the heating element, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e-liquid and is not intended to be refilled by an end user. One subset of closed system vaping smoking substitute devices include a base unit which includes the power source, wherein the base unit is configured to be physically and electrically coupled to a consumable including the tank and the heating element. The consumable may also be referred to as a cartomizer. In this way, when the tank of a consumable has been emptied, the consumable is disposed of. The base unit can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu(RTM) e-cigarette. The myblu(RTM) e-cigarette is a closed system device which includes a base unit and a consumable. The base unit and consumable are physically and electrically coupled together by pushing the consumable into the base unit. The base unit includes a rechargeable battery. The consumable includes a mouthpiece, a sealed tank which contains e-liquid, as well as a heating element, which for this device is a heating filament coiled around a portion of a wick. The wick is partially immersed in the e-liquid, and conveys e-liquid from the tank to the heating filament. The device is activated when a microprocessor on board the base unit detects a user inhaling through the mouthpiece. When the device is activated, electrical energy is supplied from the power source to the heating element, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the vaping approach, e-liquid is heated by a heating device to produce an aerosol/vapourwhich is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

In aerosol delivery devices, it is desirable to avoid leakage of aerosol precursor.

The present invention has been devised in light of the above considerations.

Document US 2017/367407 A1 discloses a disposable cartridge for use in an electronic nicotine delivery system having an electric heater.

Document WO 2017/108268 A1 discloses an electrically operated aerosol-generating system comprising: a liquid reservoir; an air inlet valve; a vapouriser configured to vaporise the liquid; a pump configured to pump liquid from the liquid reservoir to the vapouriser.

Document US 2016/345630 A1 discloses an aerosol-generating system including a liquid storage portion including a rigid housing holding a liquid aerosol-forming substrate, the housing having an opening and a fluid permeable heater assembly.

Document US 2018/220707 A1 discloses an electronic smoking device including a battery portion, an atomizer/liquid reservoir portion having a mouthpiece opening in an end, a liquid reservoir for accommodating a liquid, and a glass capillary tube.

Document US 2017/281883 A1 discloses an exemplary liquid tank for an atomizer including a bottom part, a liquid outlet, a first air inlet, a first porous liquid blocking element, and a top part.

Document US 2016/366944 A1 discloses a vaporizing apparatus includes a mouthpiece, battery and a reservoir housing an intake tube forming an air passage and vaporizing chamber proximal the battery.

Document US 2018/297047 A1 discloses a liquid supply for an electronic smoking device having a liquid reservoir within a housing for holding a liquid.

Document CN 207 011 690 U discloses an electronic cigarette atomizer, capillary including oil cup, a heat-insulating material and an intercommunication oil cup inner chamber.

Document CN 206 978 764 U discloses an electronic cigarette atomizer, capillary including oil cup, a heating element and an intercommunication oil cup inner chamber.

### Summary of the Invention

The invention is defined by the claims.

Generally, the present invention relates to an aerosol delivery device in which aerosol precursor from a liquid transfer element forms an obstruction in an air bleed channel to reduce flow through the air bleed channel.

According to the present invention, there is provided aerosol delivery device comprising:
a storage for storing aerosol precursor liquid, the storage comprising an air bleed channel for permitting air to enter the storage as the storage empties of aerosol precursor in use;
an aerosol generator for generating an aerosol from the aerosol precursor liquid for inhalation by a user; and
a liquid transfer element for transferring aerosol precursor liquid from the storage to the aerosol generator,
characterised in that the air bleed channel and the liquid transfer element are configured such that aerosol precursor liquid from the liquid transfer element forms an obstruction in the air bleed channel in use to reduce flow through the air bleed channel,
wherein the aerosol delivery device is further configured such that the obstruction is removed to open the air bleed channel in response to a user drawing on the aerosol delivery device by causing the aerosol precursor liquid in the liquid transfer element to reduce and the obstruction to be pulled from the air bleed channel into the storage to open the air bleed channel.

The air bleed channel and the liquid transfer element may be positioned such that a meniscus forms between them in use, the meniscus providing the obstruction. When the user draws on the aerosol delivery device, the aerosol generator forms an aerosol from aerosol precursor liquid, which causes the amount of aerosol precursor liquid in the liquid transfer element to reduce. The liquid transfer element then absorbs further aerosol precursor liquid from the storage, which causes the obstruction to be pulled from the air bleed channel and into the storage. This temporarily opens the air bleed channel and permits air to enter the storage to reduce the pressure difference between the storage and the external environment. Once the liquid transfer element reaches a certain level of saturation with liquid, the obstruction forms again, closing the air bleed channel.

The air bleed channel being closed prevents further liquid being transferred through the liquid transfer element until the user draws on the device again, reducing leakage. Additionally, the obstruction may prevent leakage of liquid through the air bleed channel.

Optional features will now be set out. These are applicable singly or in any combination with any aspect. The aerosol generator may comprise an aerosol generator portion configured to receive the aerosol precursor from the storage, the aerosol delivery device further comprising an air flow passage configured to direct air past the aerosol generator portion to pick up the aerosol precursor from the aerosol generator portion to form an aerosol.

The aerosol delivery device may further comprise a member, the member comprising the liquid transfer element and the aerosol generator portion

An external opening of the air bleed channel may have a diameter of less than 1 mm. The external opening may have a diameter of substantially 0.5 mm

The external opening of the air bleed channel may be located adjacent to the liquid transfer element. The liquid transfer element may define a side of the air bleed channel.

The air bleed channel may follow a tortuous path. The tortuous path may be implemented by the air bleed channel following a non-linear path, for example by turning away from the liquid transfer element.

The aerosol delivery device may further comprise a sealing element for inhibiting flow through the air bleed channel when in a deactivated state, wherein the sealing element is openable into an activated state to permit air flow through the air bleed channel when the obstruction is removed from the air bleed channel.

The sealing element may comprise a bung received in the air bleed channel in the deactivated state, the bung movable into the activated state.

The bung may comprise an enlarged portion and a neck portion, wherein the enlarged portion extends fully across the air bleed channel to block the air bleed channel in the deactivated state, and the enlarged portion moves out of the air bleed channel and the neck portion moves into the air bleed channel when the bung is moved from the deactivated to the activated state, the neck portion extending partially across the air bleed channel.

The aerosol delivery device may further comprise a barrier arrangement for inhibiting flow of aerosol precursor from the storage to the liquid transfer element, wherein the barrier arrangement is openable so that the liquid transfer element can receive aerosol precursor from the storage.

The liquid transfer element may be movable to contact the barrier arrangement to open the barrier arrangement.

The aerosol delivery device may be a consumable for a vaping device.

The aerosol delivery device may further comprise an additional aerosol generator, the additional aerosol generator configured to produce an additional aerosol from an additional aerosol precursor.

The additional aerosol generator may be configured to heat the additional aerosol precursor to form the additional aerosol.

The liquid transfer element may be configured to transfer aerosol precursor to a heated vaporiser.

The aerosol delivery device may comprise a vapour flow passage for fluid flow therethrough. The vapour flow passage may extend in a longitudinal direction between (and may fluidly connect) an inlet of the aerosol delivery device to an outlet aperture of the aerosol delivery device. The inlet may define an upstream end of the vapour flow passage, whilst the outlet aperture may define a downstream end of the vapour flow passage. The outlet aperture may be at a mouthpiece of the device and may therefore hereinafter be described as a mouthpiece aperture. In this respect, a user may draw fluid (e.g. air) into and through the vapour flow passage of the aerosol delivery device by inhaling at the mouthpiece aperture.

The terms "upstream" and "downstream" are used with reference to the direction of airflow (from inlet to outlet) through the device during normal use of the device (i.e. by way of inhalation at the mouthpiece aperture).

The vapour flow passage may comprise a plurality of passage branches that each define a separate airflow path through the aerosol delivery device. For example, in one embodiment, the aerosol delivery device may comprise first and second passage branches that are spaced laterally so as to extend along opposite lateral sides of the aerosol delivery device. The first and second passage branches may branch (e.g. in a transverse/radial direction) proximate to the vapour passage inlet, and may merge (i.e. re-join) proximate to the mouthpiece aperture.

In other embodiments, at least a portion of the vapour flow passage may comprise an annular transverse-cross sectional shape.

The aerosol delivery device may comprise a tank defining a storage chamber for containing a first aerosol precursor (e.g. a flavour liquid). The first aerosol precursor may, for example, comprise a flavourant having a menthol, liquorice, chocolate, fruit flavour (including e.g. citrus, cherry etc.), vanilla, spice (e.g. ginger, cinnamon) and/or tobacco flavour.

The first aerosol precursor may be stored in the form of a free liquid. Alternatively, a porous body may be disposed within the storage chamber, which may contain the first aerosol precursor.

The tank may at least partially define the vapour flow passage. For example, where the vapour flow passage comprises first and second passage branches, the first and second passage branches may be defined between an outer surface of the tank and an inner surface of a housing of the aerosol delivery device (i.e. a space may be formed between the tank and the housing for flow of vapour therethrough). Similarly, where at least a portion of the vapour flow passage is annular, the annular portion of the vapour flow passage may be defined between the tank and the housing.

The air bleed channel is configured to allow the bleeding of air into the storage chamber to replace (first) aerosol precursor that is removed from the storage chamber. The air bleed channel may be in fluid communication with the vapour flow passage, such that (e.g. under certain conditions) air from the vapour flow passage can enter the storage chamber through the air bleed channel.

The aerosol delivery device may comprise an aerosol generator in the form of a porous liquid transfer element (i.e. formed of a porous material). As will be described further below, the liquid transfer element may be configured to generate an aerosol in the vapour flow passage. The liquid transfer element, however, may do this in such a way that does not use heat to form the aerosol, and therefore in some embodiments may be referred to as a "passive" aerosol generator.

The liquid transfer element may comprise a conveying portion and an aerosol generating portion. The conveying portion may be elongate and generally cylindrical, and may be at least partially enclosed within one or more internal walls of the aerosol delivery device. The one or more internal walls enclosing the conveying portion may form part of the tank defining the storage chamber. In this respect, the tank may at least partly surround (e.g. may fully surround) the conveying portion of the liquid transfer element. That is, the tank may define a conduit through which the conveying portion passes. Thus, the conveying portion may extend generally longitudinally (e.g. centrally) through a portion of the tank (i.e. through the conduit defined by the tank).

The liquid transfer element may be supported in the aerosol delivery device by the mouthpiece. That is, the mouthpiece may comprise a holder for holding (and gripping) the liquid transfer element in position within the aerosol delivery device.

The aerosol generating portion of the liquid transfer element may be disposed at a downstream end of the conveying portion and may thus define a downstream longitudinal end of the liquid transfer element. The aerosol generating portion may be at least partly located in the vapour flow passage so as to be exposed to airflow within the vapour flow passage. In particular, the aerosol generating portion of the liquid transfer element may extend into an aerosolisation chamber forming part of the vapour flow passage. The aerosolisation chamber may be located proximate to (and in fluid communication with) the mouthpiece aperture of the device and may define a portion of the vapour flow passage at the first and second passage branches of the vapour flow passage merge. Airflow through the flow passage may pass across or through the aerosol generating portion of the liquid transfer element prior to being discharged through the mouthpiece aperture.

The aerosol generating portion may define an enlarged (e.g. radially enlarged) portion of the liquid transfer element. For example, the aerosol generating portion may be bulb-shaped or bullet-shaped, and may comprise a portion which is wider than the conveying portion. The aerosol generating portion may taper (inwardly) to a tip at a downstream end of the aerosol generating portion (i.e. proximate the outlet/mouthpiece aperture). The aerosol-generating portion may have a flattened downstream end surface.

The liquid transfer element may extend into the storage chamber so as to be in contact with (e.g. at least partially submerged in) the first aerosol precursor. In this way, the liquid transfer element may be configured to convey (e.g. via a wicking/capillary action) the first aerosol precursor from the storage chamber to the aerosolisation chamber. As will be described further below, this may allow the first aerosol precursor to form an aerosol and be entrained in an airflow passing through aerosolisation chamber (i.e. for subsequent receipt in a user's mouth).

The vapour flow passage may be constricted (i.e. narrowed) at the aerosolisation chamber. For example, the presence of the aerosol generating portion in the vapour flow passage may create a constricted or narrowed portion of the vapour flow passage (because the aerosol generating portion extends partway across the vapour flow passage). In this respect, the narrowest portion of the vapour flow passage may be at aerosolisation chamber (adjacent to the aerosol generating portion of the liquid transfer element). This constriction of the vapour flow passage increases the velocity of air/vapour passing through the aerosolisation chamber. In this respect, the constriction may be referred to as a Venturi aperture. The constriction may have a toroidal shape (i.e. extending about the aerosol generating portion of the liquid transfer element). The toroidal shape may, however, be interrupted by supports (e.g. projections, ribs, etc.) protruding inwardly from wall(s) of the vapour flow passage to support the aerosol generating portion in the aerosolisation chamber.

In addition to increasing the airflow velocity, the constriction reduces the air pressure of the airflow flowing through the constriction (i.e. in the vicinity of the aerosol generating portion). This low pressure and high velocity facilitate the generation of an aerosol from the first aerosol precursor held in the aerosol generating portion (i.e. transferred from the storage chamber by the liquid transfer element). This aerosol, which is hereinafter referred to as the first aerosol, is entrained in the airflow passing through the constriction and is discharged from the mouthpiece aperture of the aerosol delivery device.

In other examples the liquid transfer element does not comprise an aerosol generator, and is instead configured to transfer liquid, for example to a separate aerosol generator or vaporiser.

The first aerosol may be sized to inhibit pulmonary penetration. The first aerosol may be formed of particles with a mass median aerodynamic diameter that is greater than or equal to 15 microns, e.g. greater than 30 microns, or greater than 50 microns, or may be greater than 60 microns, or may be greater than 70 microns.

The first aerosol may be sized for transmission within at least one of a mammalian oral cavity and a mammalian nasal cavity. The first aerosol may be formed by particles having a maximum mass median aerodynamic diameter that is less than 300 microns, or e.g. less than 200 microns, or less than 100 microns. Such a range of mass median aerodynamic diameter can produce aerosols which are sufficiently small to be entrained in an airflow caused by a user drawing air through the aerosol delivery device and to enter and extend through the oral and or nasal cavity to activate the taste and/or olfactory receptors.

The size of aerosol formed without heating may be typically smaller than that formed by condensation of a vapour.

It is noted that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

The above configuration of the aerosol delivery device may be representative of an activated state of the aerosol delivery device. The aerosol delivery device may additionally be configurable in a deactivated state. In the deactivated state, the liquid transfer element may be isolated from the first aerosol precursor. This isolation may, for example, be provided by a plug (e.g. formed of silicon). The plug may be located at an end (i.e. upstream end) of the conduit (defined by the tank) so as to provide a barrier between the first aerosol precursor in the storage chamber and the conveying portion of the liquid transfer element. Alternatively, the aerosol delivery device may comprise a duck bill valve, a split valve or diaphragm; or a sheet of foil isolating the liquid transfer element from the first aerosol precursor.

In the deactivated state, the air bleed channel may be sealed by a sealing element. The sealing element may, for example, be in the form of a bung or plug (e.g. a silicone bung or plug). At least a portion of the bung may be received in the air bleed channel when the aerosol delivery device is in the deactivated state, so as to block the passage of airflow through the air bleed channel. The sealing element may alternatively be in the form of a pierceable membrane (e.g. formed of a metal foil) extending across the air bleed channel.

The aerosol delivery device may comprise a terminal component (e.g. the mouthpiece) that is movable relative to the tank defining the storage chamber. The terminal component/mouthpiece may be movable relative to the air bleed channel. In particular, movement of the terminal component may be in the longitudinal direction of the aerosol delivery device.

The terminal component/mouthpiece may comprise an activation member, which may protrude internally from an internal surface of terminal component/mouthpiece. When the terminal component/mouthpiece is moved longitudinally in an upstream direction i.e. towards the storage tank, a distal end of the activation member may engage the sealing element so as to move the sealing element (i.e. in the upstream direction) relative to the air bleed channel. This movement of the sealing element may open the air bleed channel, so as to allow airflow therethrough and so as to move the aerosol delivery device to the activated state.

When the sealing element is a bung, the bung may comprise an enlarged portion, in some cases an enlarged end that extends fully across the air bleed channel, and a neck portion that extends only partway across the air bleed channel. Movement of the bung along the air bleed channel by the activation member may cause the enlarged end of the bung to move into the storage chamber such that only the neck portion remains in the air bleed channel. Thus, airflow may be permitted through the air bleed channel between the neck portion and the walls of the air bleed channel.

When the sealing element is a pierceable membrane, the activation member may pierce the pierceable membrane when moved in the upstream direction. To facilitate such piercing, the activation member may be in the form of a blade, or may be pointed.

The movement of the terminal component (e.g. the mouthpiece) may also cause longitudinal upstream movement of the liquid transfer element through the conduit defined by the tank. The conveying portion of the liquid transfer element may engage the plug (or duck bill valve, split valve, etc.) so as to disengage the plug from the end of the conduit. Removal of the plug in this way means that the conveying portion comes into contact with the first aerosol precursor (i.e. so as to be able to convey the first aerosol precursor to the aerosol generating portion of the liquid transfer element).

The above components of the aerosol delivery device may form a consumable portion of the aerosol delivery device, and may collectively be referred to as an additive delivery article or flavour pod of the aerosol delivery device.

The aerosol delivery device may further comprise a cartomizer. The additive delivery article/flavour pod may be engageable with the cartomizer, for example, by way of an interference fit, snap-engagement, bayonet locking arrangement, etc. In other embodiments, the additive delivery article/flavour pod and cartomizer may be integrally formed (e.g. defining a single consumable article).

The cartomizer may comprise a vaporising chamber and a vaporiser outlet for fluid flow therethrough. The vaporiser outlet may be fluidly connected to the vapour flow passage of the additive delivery article/flavour pod. The vaporiser outlet and vaporising chamber may fluidly connect a cartomizer inlet opening and the inlet of the vapour flow passage. Thus, an airflow may be drawn into and through the cartomizer, and subsequently through the additive delivery article/flavour pod.

The aerosol delivery device may comprise a reservoir defined by a container for containing a second aerosol precursor (which may be an e-liquid). The second aerosol precursor may, for example, comprise a base liquid and a physiologically active compound e.g. nicotine. The base liquid may include an aerosol former such as propylene glycol and/or vegetable glycerine.

At least a portion of the container may be translucent. For example, the container may comprise a window to allow a user to visually assess the quantity of second aerosol precursor in the container. The cartomizer may be referred to as a "clearomizer" if it includes a window. The vaporiser outlet may extend longitudinally through the container, wherein an outlet wall of the vaporiser outlet may define the inner wall of the container. In this respect, the container may surround the vaporiser outlet, such that the container may be generally annular.

The aerosol delivery device may comprise a vaporiser. The vaporiser may be located in the vaporising chamber.

The vaporiser may comprise a wick. The vaporiser may further comprise a heater. The wick may comprise a porous material. A portion of the wick may be exposed to fluid flow in the vaporising chamber. The wick may also comprise one or more portions in contact with the second aerosol precursor stored in the reservoir. For example, opposing ends of the wick may protrude into the reservoir and a central portion (between the ends) may extend across the vaporising chamber so as to be exposed to air flow in the vaporising chamber. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid/air flowing through the vaporising chamber. This vapour may subsequently cool to form an aerosol in the vaporiser outlet. This aerosol is hereinafter referred to as the second aerosol. This aerosol generation may be referred to as "active" aerosol generation, because it makes use of heat to generate the aerosol.

This second aerosol may subsequently flow from the vaporiser outlet to (and through) the vapour flow passage of the additive delivery article/flavour pod (e.g. when engaged with the cartomizer). Thus, the fluid received through the mouthpiece aperture of the aerosol delivery device may be a combination of the first aerosol and the second aerosol.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The cartomizer may be a consumable part of the aerosol delivery device. For example, the cartomizer may be configured for engagement with a base unit. The cartomizer and the additive delivery article/flavour pod may be a single consumable component of the aerosol delivery device (when integrally formed) or may each define separate consumable components of the aerosol delivery device (when engageable with one another).

Thus, the cartomizer and additive delivery article/flavour pod (whether integral or separable) may define, and will be referred to herein as, a consumable of the aerosol delivery device. The first aerosol precursor and second aerosol precursor may be replenished by replacing a used consumable with an unused consumable. It should be appreciated that some of the features described herein as being part of the consumable may alternatively form part of a base unit for engagement with the consumable.

The base unit and the consumable (e.g. the cartomizer of the consumable) may be configured to be physically coupled together. For example, the consumable may be at least partially received in a recess of the base unit, such that there is snap engagement between the base unit and the consumable. Alternatively, the base unit and the consumable may be physically coupled together by screwing one onto the other, or through a bayonet fitting.

Thus, the consumable may comprise one or more engagement portions for engaging with a base unit. In this way, one end of the consumable (i.e. the inlet end) may be coupled with the base unit, whilst an opposing end (i.e. the outlet end) of the consumable may define the mouthpiece.

The base unit or the consumable may comprise a power source or be connectable to a power source. The power source may be electrically connected (or connectable) to the heater. The power source may be a battery (e.g. a rechargeable battery). An external electrical connector in the form of e.g. a USB port may be provided for recharging this battery.

The consumable may comprise an electrical interface for interfacing with a corresponding electrical interface of the base unit. One or both of the electrical interfaces may include one or more electrical contacts. Thus, when the base unit is engaged with the consumable, the electrical interface may be configured to transfer electrical power from the power source to a heater of the consumable. The electrical interface may also be used to identify the consumable from a list of known types. The electrical interface may additionally or alternatively be used to identify when the consumable is connected to the base unit.

The base unit may alternatively or additionally be able to detect information about the consumable via an RFID reader, a barcode or QR code reader. This interface may be able to identify a characteristic (e.g. a type) of the consumable. In this respect, the consumable may include any one or more of an RFID chip, a barcode or QR code, or memory within which is an identifier and which can be interrogated via the interface.

The consumable or base unit may comprise a controller, which may include a microprocessor. The controller may be configured to control the supply of power from the power source to the heater (e.g. via the electrical contacts). A memory may be provided and may be operatively connected to the controller. The memory may include non-volatile memory. The memory may include instructions which, when implemented, cause the controller to perform certain tasks or steps of a method.

The consumable or base unit may comprise a wireless interface, which may be configured to communicate wirelessly with another device, for example a mobile device, e.g. via Bluetooth^{®}. To this end, the wireless interface could include a Bluetooth^{®} antenna. Other wireless communication interfaces, e.g. WiFi^{®}, are also possible. The wireless interface may also be configured to communicate wirelessly with a remote server.

An airflow (i.e. puff) sensor may be provided that is configured to detect a puff (i.e. inhalation from a user). The airflow sensor may be operatively connected to the controller so as to be able to provide a signal to the controller that is indicative of a puff state (i.e. puffing or not puffing). The airflow sensor may, for example, be in the form of a pressure sensor or an acoustic sensor. The controller may control power supply to the heater in response to airflow detection by the sensor. The control may be in the form of activation of the heater in response to a detected airflow. The airflow sensor may form part of the consumable (e.g. the cartomizer or additive delivery article/flavour pod) or the base unit.

In an alternative embodiment the aerosol delivery device may be a non-consumable device in which one or both of the first and second aerosol precursors of the device may be replenished by re-filling the reservoir or storage chamber of the device (rather than replacing the consumable). In this embodiment, the consumable described above may instead be a non-consumable component that is integral with the base unit. Thus the device may comprise the features of the base unit described above. For example, the only consumable portion may be first and/or second aerosol precursor contained in reservoir and storage chamber of the device. Access to the reservoir and/or storage chamber (for re-filling of the aerosol precursor) may be provided via e.g. an opening to the reservoir and/or storage chamber that is sealable with a closure (e.g. a cap).

The aerosol delivery device may be a smoking substitute device (e.g. an e-cigarette device). The consumable of the aerosol delivery device be a smoking substitute consumable (e.g. an e-cigarette consumable).

In a second example there is provided an aerosol delivery device comprising:
a storage for storing aerosol precursor liquid, the storage comprising an air bleed channel for permitting air to enter the storage as the storage empties of aerosol precursor in use;
an aerosol generator for generating an aerosol from the aerosol precursor liquid for inhalation by a user; and
a liquid transfer element for transferring aerosol precursor liquid from the storage to an aerosol generator,
wherein the air bleed channel and the liquid transfer element are configured such that aerosol precursor liquid from the liquid transfer element forms an obstruction in the air bleed channel in use to reduce flow through the air bleed channel, and
the aerosol delivery device is further configured such that the obstruction is removed to open the air bleed channel in response to a user drawing on the aerosol delivery device by causing the aerosol precursor liquid in the liquid transfer element to reduce and the obstruction to be pulled from the air bleed channel into the storage to open the air bleed channel.

The aerosol delivery device of the second aspect may otherwise be as described above with respect to the first aspect.

In a third example there is provided a smoking substitute system comprising a base unit having a power source, and a consumable as described above with respect to the first or second aspect, the consumable being engageable with the base unit such that a vaporiser of the consumable is connected to the power source of the base unit. The consumable may comprise a cartomizer and an additive delivery article/flavour pod, each as described above with respect to the first or second aspect. The cartomizer and additive delivery article/flavour pod may be integrally formed, or may be separate, but engageable with one another.

In a fourth example there is provided a method of using a smoking substitute system as described above with respect to the third aspect, the method comprising engaging the consumable with the base unit so as to connect the vaporiser of the consumable with the power source of the base unit.

The method may comprise engaging an additive delivery article/flavour pod of the consumable with a cartomizer of the consumable, such that a vapour flow passage of the additive delivery article/flavour pod is in fluid communication with the vaporiser outlet of the cartomizer.

### Summary of the Figures

So that the invention may be understood, and so that further aspects and features thereof may be appreciated, embodiments illustrating the principles of the invention or aiding in the understanding of the invention will now be discussed in further detail with reference to the accompanying figures, in which:
Figures 1A and 1B is a schematic drawing of an aerosol delivery device according to a first embodiment;
Figures 2A and 2B is a schematic drawing of an aerosol delivery device according to a second embodiment;
Figure 3A is a cross-sectional view of a consumable, according to a third embodiment, in a deactivated state;
Figure 3B is a cross-sectional view of the consumable of Figure 3A in an activated state;
Figure 3C is a cross-sectional schematic view of the flavour pod portion of the consumable of the third embodiment;
Figures 3D and 3E are respective top and perspective views of a mouthpiece of the third embodiment;
Figure 4A is a cross-sectional view of a consumable, according to a fourth embodiment, in a deactivated state; and
Figure 4B is a cross-sectional view of a consumable, according to a fourth embodiment, in an activated state.

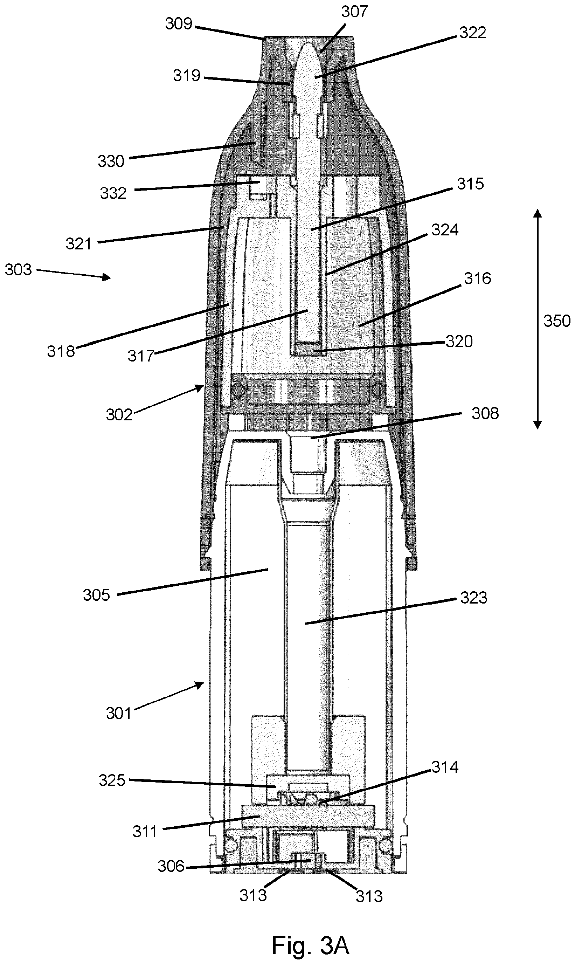

The examples shown in Figures 3A to 3E do not illustrate the invention, but are provided to aid in the understanding of the invention.

### Detailed Description of the Invention

Aspects and embodiments of the present invention will now be discussed with reference to the accompanying figures. Further aspects and embodiments will be apparent to those skilled in the art.

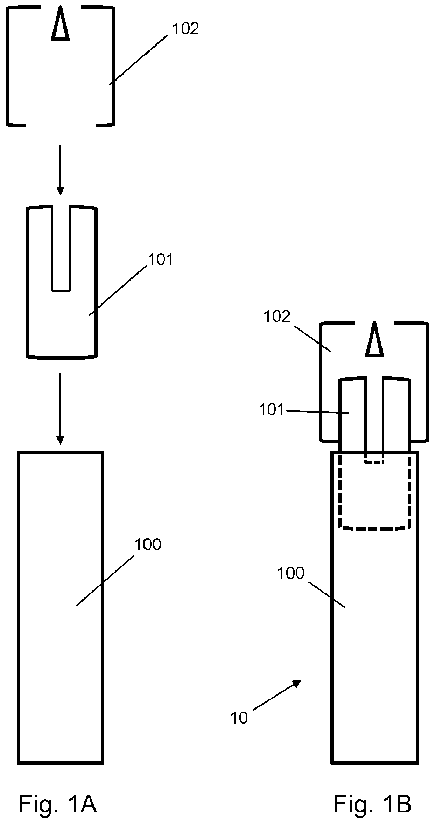

Referring to figures 1A and 1B, there is shown an schematic view of an aerosol delivery device in the form of a smoking substitute device 10. In this example, the smoking substitute device 10 comprises a cartomizer 101 and an additive delivery article in the form of a flavour pod 102 connected to a base unit 100. In this example, the base unit 100 includes elements of the smoking substitute device 10 such as a battery, an electronic controller, and a pressure transducer (not shown). The cartomizer 101 may engage with the base unit 100 via a push-fit engagement, a screw-thread engagement, or a bayonet fit, for example. A cartomizer may also be referred to as a "pod".

The flavour pod 102 is configured to engage with the cartomizer 101 and thus with the base unit 100. The flavour pod 102 may engage with the cartomizer 101 via a push-fit engagement, a screw-thread engagement, or a bayonet fit, for example. Figure 1B illustrates the cartomizer 101 engaged with the base unit 100, and the flavour pod 102 engaged with the cartomizer 101. As will be appreciated, in this example, the cartomizer 101 and the flavour pod 102 are distinct elements.

As will be appreciated from the following description, in other embodiments the cartomizer 101 and the flavour pod 102 may be combined into a single component that implements the combined functionality of the cartomizer 101 and flavour pod 102. Such a single component may also be referred to as an aerosol delivery device. In other examples, the cartomizer may be absent, with only a flavour pod 102 present.

As is set forth above, reference to a "consumable" component may mean that the component is intended to be used once until exhausted, and then disposed of as waste or returned to a manufacturer for reprocessing.

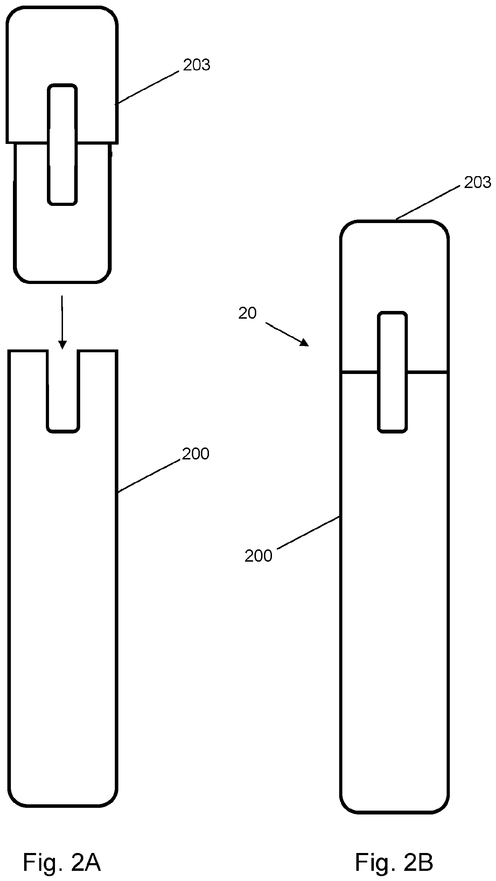

Referring to figures 2A and 2B, there is shown a smoking substitute device 20 comprising a base unit 200 and a consumable 203. The consumable 203 combines the functionality of the cartomizer 201 and the flavour pod 202. In Figure 2A, the consumable 203 and the base unit 200 are shown separated from one another. In Figure 2B, the consumable 203 and the base unit 200 are engaged with each other to form the smoking substitute device 20.

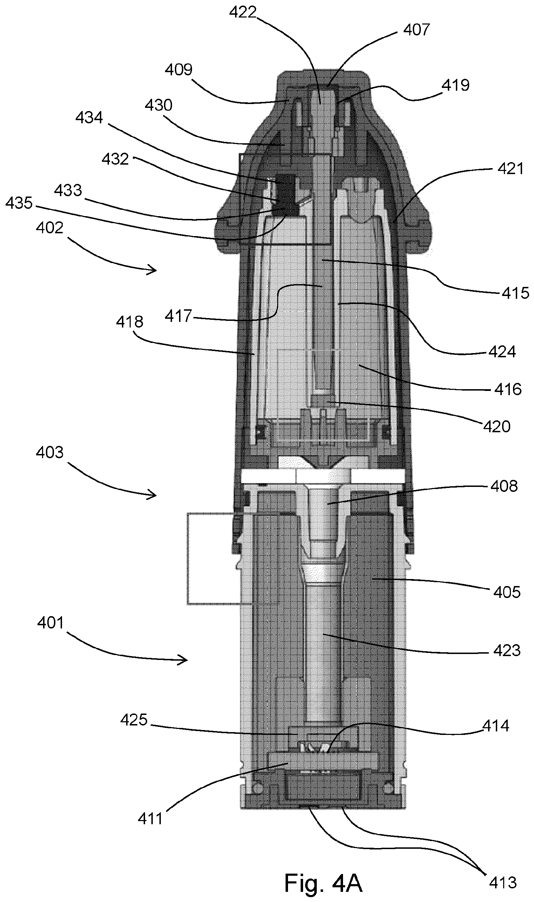

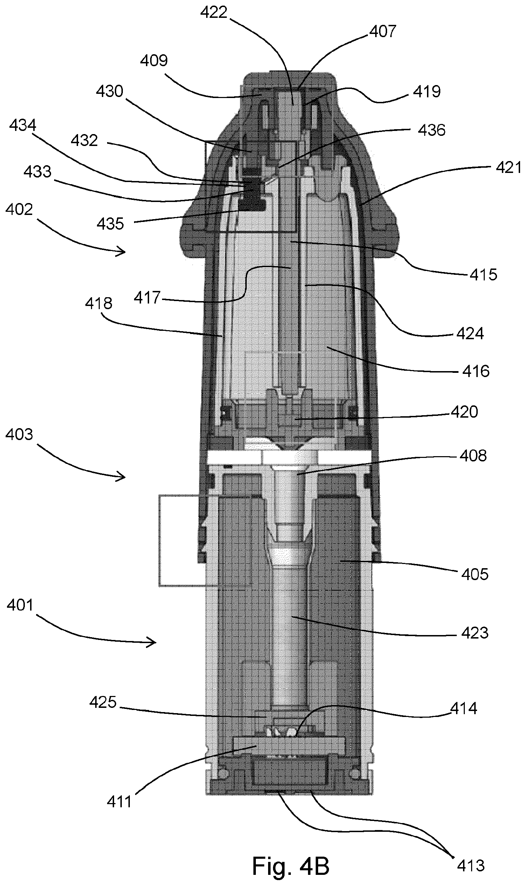

The examples shown in Figures 3A to 3E do not illustrate the invention, but are provided to aid in the understanding of the invention described below with reference to Figures 4A and 4B.

Referring to Figure 3A, there is shown a consumable 303 engagable with a base unit (not shown) via a push-fit engagement. The consumable 303 is shown in a deactivated state. The consumable 303 may be considered to have two portions - a cartomizer portion 301 and flavour pod portion 302 (i.e. additive delivery device), both of which are located within a single consumable component 303 (as in figures 2A and 2B). It should, however, be appreciated that in a variation, the cartomizer portion 301 and flavour pod portion 302 may be separate (but engageable) components.

The consumable 303 includes an upstream cartomizer inlet opening 306 and a downstream mouthpiece aperture 307 (i.e. defining an outlet of the consumable 303). In other examples a plurality of inlets and/or outlets are included. Between, and fluidly connecting, the inlet opening 306 and the mouthpiece aperture 307 there is an airflow passage 308. This airflow passage 308 is formed (in a downstream flow direction) of a vaporising chamber 325 of the cartomizer, a vapour outlet 323 (also of the cartomizer) and a downstream vapour flow passage 321 of the flavour pod portion 302. The mouthpiece aperture 307 is located at the mouthpiece 309 of the consumable 303.

As above, the consumable 303 includes a flavour pod portion 302. The flavour pod portion 302 is configured to generate a first (flavour) aerosol for output from the mouthpiece aperture 307. The flavour pod portion 302 of the consumable 303 includes a liquid transfer element 315. This liquid transfer element 315 acts as a passive aerosol generator (i.e. an aerosol generator which does not use heat to form the aerosol, also referred to as a "first aerosol generator" herein), and is formed of a porous material. The liquid transfer element 315 comprises a conveying portion 317 and an aerosol generating portion 322, which is located in the vapour flow passage 321. In this example, the aerosol generating portion 322 is a porous nib.

When activated, as discussed in more detail below, a storage chamber 316 (defined by a tank 318) for storing a first aerosol precursor (i.e. a liquid comprising a flavourant) is fluidly connected to the liquid transfer element 315. The first aerosol precursor, in this embodiment, is stored in a porous body within the storage chamber 316 (but may be a free-liquid). In the activated state, the liquid transfer element 315 is in contact with the first aerosol precursor stored in the storage chamber 316 by way of contact with the porous body/free liquid.

The liquid transfer element 315 comprises an aerosol generating portion 322 and a conveying portion 317. The aerosol generating portion 322 is located at a downstream end (top of Figure 3A) of the liquid transfer element 315, whilst the conveying portion 317 forms the remainder of the liquid transfer element 315. The conveying portion 317 is elongate and substantially cylindrical. The aerosol generating portion 322 is bulb/bullet-shaped, and comprises a portion which is wider (has a greater radius) than the conveying portion 317. The aerosol generating portion 322 tapers to a tip at a downstream end of the liquid transfer element 315. In other examples the liquid transfer element 315 comprises a conveying portion but not an aerosol generating portion, and the conveying portion is configured to transfer liquid to a separate aerosol generator.

The liquid transfer element 315 extends into and through the storage chamber 316, such that the conveying portion 317 is in contact with the contents of the storage chamber 316. In particular, an inner wall of the tank 318 defines a conduit 324, through which the liquid transfer element 315 extends. The liquid transfer element 315 and the conduit 324 are located in a substantially central position within the storage chamber 216 and are substantially parallel to a central longitudinal axis of the consumable 303.

The porous nature of the liquid transfer element 315 means that first aerosol precursor in the storage chamber 316 is drawn into the liquid transfer element 315. As the first aerosol precursor in the liquid transfer element 315 is depleted in use, further aerosol precursor is drawn from the storage chamber 316 into the liquid transfer element 315 via a wicking action.

Before activation, the storage chamber 316 is fluidly isolated from the liquid transfer element 315 by a barrier arrangement. In this example, the isolation is achieved via a plug 320 (preferably formed from silicone) located at one end of a conduit 324 surrounding the liquid transfer element 315. In other examples, the plug may be replaced by any one of: a duck bill valve; a split valve or diaphragm; or a sheet of foil.

The storage chamber 316 further includes an air bleed channel 332, which in the deactivated state is sealed by a pierceable membrane (preferably made from foil). Activation (or piercing) member 330, which projects inwardly from the mouthpiece 309, and may take the form of a blade, pierces the pierceable membrane and opens the air bleed channel 332 when the consumable is moved to the activated state (as is discussed in more detail below).

The aerosol generating portion 322 is located within the vapour flow passage 321 that extends through the flavour pod portion 302. The aerosol generating portion 322, by occupying a portion of the vapour flow passage 321, constricts or narrows the vapour flow passage 321. This constricted or narrowed portion of the vapour flow passage 321 defines an aerosolisation chamber 319 of the consumable 303. The aerosolisation chamber 319, which is adjacent the aerosol generating portion 322, is the narrowest portion of the vapour flow passage 321. The constriction of the vapour flow passage 321 at the aerosolisation chamber 319 results in increased air velocity and a corresponding reduction in air pressure of the air flowing therethrough and thus may be referred to as a Venturi aperture. The aerosolisation chamber 319 is generally toroidal in shape (extending circumferentially about the aerosol generating portion 322), but this toroidal shape may include one or more interruptions where supports extend inwardly to contact the aerosol generating portion 322 and to support the aerosol generating portion 322 within the aerosolisation chamber 319.

The cartomizer portion 301 of the consumable 303 includes a reservoir 305 (defined by a container) for storing a second aerosol precursor (i.e. e-liquid, which may contain nicotine). A wick 311 extends into the reservoir so as to be in contact with (i.e. partially submerged in) the second aerosol precursor. The wick 311 is formed from a porous wicking material (e.g. a polymer) that draws the second aerosol precursor from the reservoir 305 into a central region of the wick 311 that is located in the vaporising chamber 325.

A heater 314 is a configured to heat the central region of the wick 311. The heater 314 includes a resistive heating filament that is coiled around the central region of the wick 311. The wick 311 and the heater 314 generally define a vaporiser, and together with the reservoir 305 act as an active aerosol generator. The vaporiser (i.e. wick 311 and heater 314) and aerosol generating portion 322 are both at least partially located within the airflow passage 308, with the aerosol generating portion 322 being downstream of the vaporiser.

So that the consumable 303 may be supplied with electrical power for activation of the heater 314, the consumable 303 includes a pair of consumable electrical contacts 313. The consumable electrical contacts 313 are configured for electrical connection to a corresponding pair of electrical supply contacts in the base unit (not shown). The consumable electrical contacts 313 are electrically connected to the electrical supply contacts (not shown) when the consumable 303 is engaged with the base unit. The base unit includes an electrical power source, for example a battery.

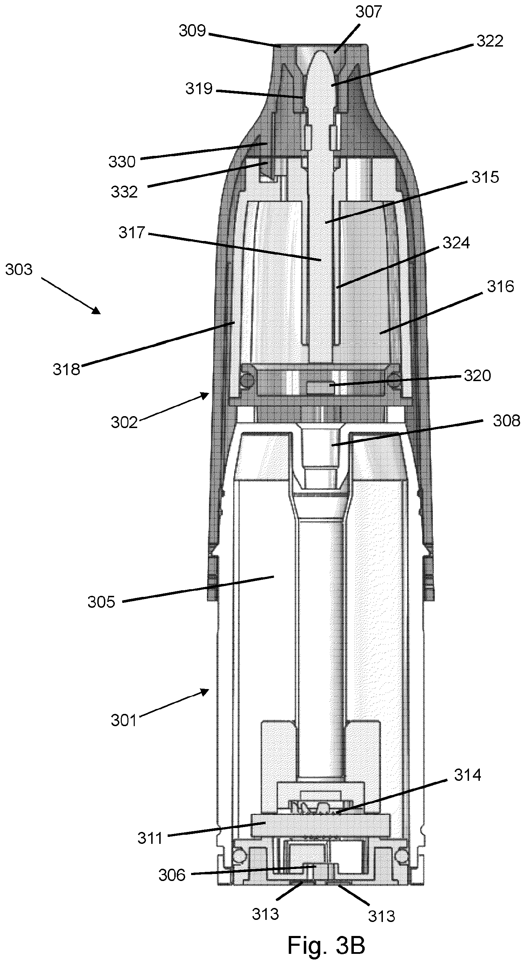

Figure 3B shows the consumable 303 of Figure 3A in an activated state. To transition from the deactivated state to the activated state, mouthpiece 309 is moved along a central longitudinal axis 350 in an upstream direction towards cartomizer portion 301. The mouthpiece 309 is fixed by a collar to the conveying portion 317 of the liquid transfer element 315 and therefore liquid transfer element 315 moves with the mouthpiece 309. The mouthpiece 309 and liquid transfer element 315 are moved relative to the tank 316.

When the mouthpiece 309 is moved upstream, activation/piercing member 330 contacts and pierces a sealing element in the form of a pierceable membrane extending across the air bleed channel 332 thereby fluidly connecting the vapour flow passage 321 the storage chamber 316. This allows air from the vapour flow passage 321 to enter the storage chamber 316 as aerosol precursor is removed from the storage chamber 316 by the liquid transfer element 315.

In addition to piercing of the membrane by the piercing member 330, liquid transfer element 315 pushes on, and moves, barrier element 320 (in the form of plug 320) out of the conduit 324 which then allows liquid transfer element 315 to come into contact with the first aerosol precursor stored in the first storage chamber 316. The plug 320 may then be unconstrained within the storage chamber, or may be pushed by liquid transfer element 315 into a holding location.

Once activated, and in use, a user draws (or "sucks", "pulls", or "puffs") on the mouthpiece 309 of the consumable 303, which causes a drop in air pressure at the mouthpiece aperture 307, thereby generating air flow through the inlet opening 306, along the airflow passage 308, out of the mouthpiece aperture 307 and into the user's mouth.

When the heater 314 is activated by passing an electric current through the heating filament in response to the user drawing on the mouthpiece 309 (the drawing of air may be detected by a pressure transducer), the e-liquid located in the wick 311 adjacent to the heating filament is heated and vaporised to form a vapour in the vaporising chamber 325. The vapour condenses to form the second aerosol within the vaporiser outlet 323. The second aerosol is entrained in an airflow along the vapour flow passage 321 to the mouthpiece aperture 307 for inhalation by the user when the user draws on the mouthpiece 309.

The base unit supplies electrical current to the consumable electrical contacts 113. This causes an electric current flow through the heating filament of the heater 314 and the heating filament heats up. As described, the heating of the heating filament causes vaporisation of the e-liquid in the wick 311 to form the second aerosol.

As the air flows through the vapour flow passage 321, it encounters the aerosol generating portion 322. The constriction of the vapour flow passage 321, at the aerosolisation chamber 319, results in an increase in air velocity and corresponding decrease in air pressure in the airflow in the vicinity of the porous aerosol generating portion 322. The corresponding low pressure and high air velocity region causes the generation of the first (flavour) aerosol from the porous surface of the aerosol generating portion 322 of the liquid transfer element 315. The first (flavour) aerosol becomes entrained in the airflow and ultimately is output from the mouthpiece aperture 307 of the consumable 303 and into the user's mouth.

The first aerosol is sized to inhibit pulmonary penetration. The first aerosol is formed of particles with a mass median aerodynamic diameter that is greater than 70 microns. The first aerosol is sized for transmission within at least one of a mammalian oral cavity and a mammalian nasal cavity. The first aerosol is formed by particles having a maximum mass median aerodynamic diameter that is less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated is sized for pulmonary penetration (i.e. to deliver an active ingredient such as nicotine to the user's lungs). The second aerosol is formed of particles having a mass median aerodynamic diameter of less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

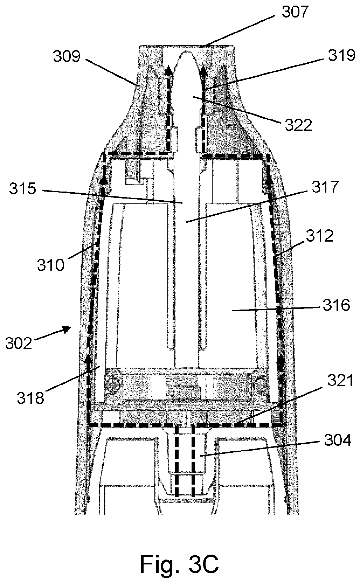

Figure 3C illustrates the flow of vapour through the flavour pod portion 302 of figures 3A and 3B. The flavour pod portion 302 is shown in the activated state. The cartomizer is not shown, but it should be appreciated that the flavour pod portions 302 is engaged with the cartomizer 301 of figures 3A and 3B. In other embodiments, however, the consumable 303 may not comprise a cartomizer portion, and may provide only flavour to the user.

As is provided above, the flavour pod portion 302 comprises an upstream (i.e. upstream with respect to flow of air in use) vapour passage inlet 304 and a downstream (i.e. downstream with respect to flow of air in use) outlet in the form of a mouthpiece aperture 307. Between, and fluidly connecting the vapour passage inlet 304 and the mouthpiece aperture 307, is a vapour flow passage 321. The vapour flow passage 321 comprises a first passage branch 310 and a second passage branch 312, each of the first passage branch 310 and the second passage branch 312 fluidly connecting the vapour passage inlet 304 and the mouthpiece aperture 307. In other examples the vapour flow passage 321 may have an annular shape.

The first and second passage branches 310, 312 are generally located on opposite sides of the liquid transfer element 315. Additionally, the first and second airflow branches 310, 312 are located on opposite sides of the storage chamber 316. The first and second airflow branches 310, 312 branch in a transverse (i.e. radially) outward direction (with respect to a central longitudinal axis of the consumable 303) downstream of the vapour passage inlet 304 to reach respective opposite sides of the storage chamber 316.

The aerosol generating portion 322 is located in the vapour flow passage 321 downstream of the first and second passage branches 310, 312. The first and second passage branches 310, 312 turn in a transverse and radially inward direction to merge at the liquid transfer element 315, and at a point upstream of the aerosol generating portion 322.

The aerosolisation chamber 319 is thus downstream of the point at which the first and second passage branches 310, 312 merge, but upstream of the mouthpiece aperture 307. A transition region, between the aerosolisation chamber 319 and the mouthpiece aperture 307 flares outwardly in the downstream direction, such that a diameter of the mouthpiece aperture 307 is greater than a diameter of the aerosolisation chamber 319.

In use, when a user draws on the mouthpiece 309, air flow is generated through the air flow passage 308. Air (comprising the second aerosol from the cartomizer portion 301 as explained above with respect to Figure 3A) flows through the vapour passage inlet 304 before the air flow splits to flow through the first and second passage branches 310, 312. Further downstream, the first and second passage branches 310, 312 provide inward airflow towards the liquid transfer element 315 and the aerosol generating portion 322.

As air flows past the aerosol generating portion 322 in the aerosolisation chamber 319, the velocity of the air increases, resulting in a drop in air pressure. As a result, the first aerosol precursor held in the aerosol generating portion 322 becomes entrained in the air so as to form the first aerosol. The first aerosol has the particle size and other properties described above with respect to Figure 3A.

As the first aerosol precursor becomes entrained within the air, the liquid transfer element 315 transfers further first aerosol precursor from the storage chamber 316 to the aerosol generating portion 322. More specifically, the liquid transfer element wicks the first aerosol precursor from the storage chamber 316 to the aerosol generating portion 322.

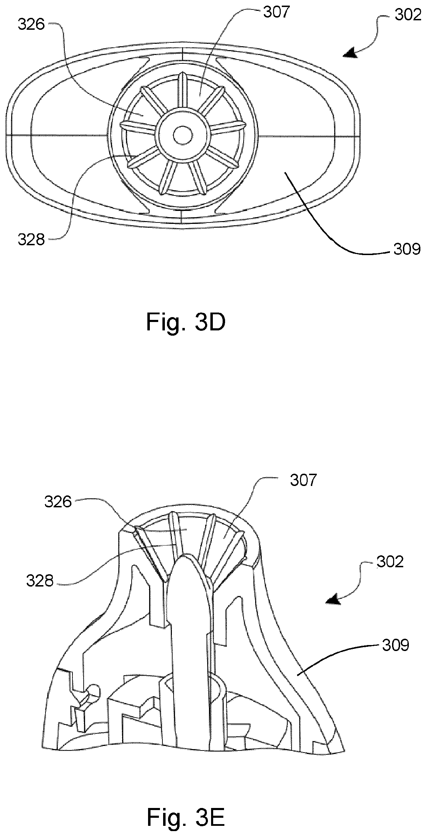

Figures 3D and 3E show further views of the flavour pod portion 302 which highlight features of the mouthpiece 309. Many of the reference numerals of Figure 3C are omitted from figures 3D and 3E for clarity.

An uneven inner (transition) surface 326 is located between the mouthpiece aperture 307 and the aerosolisation chamber 319. In the present example, the inner surface 326 has the form of a substantially frustoconical surface, but includes grooves or channels 328 to make the inner surface 326 somewhat uneven. In other examples, the inner surface 326 may have another form (for example, the form a substantially cylindrical surface), and may include any type of protrusion or groove to make the inner surface uneven.

The inner surface 326 is angled with respect to an axial direction (i.e. relative to a central axis extending from a base of the consumable to the mouthpiece) such that the diameter of the passage 321 proximate the mouthpiece aperture 307 increases in the downstream direction. The inner surface 326 is downstream of the aerosolisation chamber 319 of the vapour flow passage 321.

The grooves 328 are generally V-shaped in cross-sectional profile, and extend in the axial direction for the full length of the inner surface 326. Each groove 328 is formed from a pair of surfaces angled at between 30 and 90 degrees (e.g. 60 degrees) relative to each other. The grooves 328 have a depth (measured normal to the inner surface 326) of at least 0.2 mm (e.g. at least 0.4 mm). The grooves 328 have a depth of less than 0.8 mm (e.g. less than 0.6 mm). The grooves have a depth of substantially 0.5 mm. The inner surface 326 comprises 9 grooves 328, but may comprise more or less grooves.

The grooves 328 are spaced apart from each other by substantially 1 mm at the downstream end of the inner surface 326. In other examples, the spacing at the downstream end of grooves or protrusions may be selected such that it is equal to or less than the mass median diameter (as described above) of particles in the first aerosol.

The inner surface 326 comprises a smooth polished surface between the grooves 328. Polishing the surface in this way may provide improved aerodynamic properties. However, in other examples, the inner surface 326 may be textured. In such examples, the texture of the surface may provide the uneven surface, and no grooves may be required.

In use, the uneven nature of the inner surface 326 may make it easier for droplets to form on the inner surface 326, preventing large droplets from entering the user's mouth. The grooves 328 may help to channel the large droplets back into the consumable.

Figures 4A and 4B illustrate a further embodiment of a consumable 403 of an aerosol delivery device.

This embodiment includes many of the same features of the embodiment described above and shown in Figures 3A-3E and, for that reason, corresponding reference numerals have been used (albeit with a unit increase of the first digit to represent the further embodiment). The description of those features have not been repeated here. Figure 4A shows the consumable 403 in a deactivated state and Figure 4B shows the consumable 403 in an activated state.

Unlike the previously described embodiment, the presently illustrated embodiment comprises a different mechanism for opening the air bleed channel 432 upon activation of the consumable 403. In this embodiment, when in the deactivated state (Figure 4A) the air bleed channel 432 is blocked by a silicone bung 433 sealing element received in the air bleed channel 432. In particular, a body 434 of the bung 433 is received in the air bleed channel 432, but does not fully obstruct the air bleed channel 432. That is, a portion of the air bleed channel 432 remains unobstructed by the body 434 of the bung 433. However, an enlarged portion, in this example an enlarged head 435 of the bung 433, which is located in the storage chamber 416, extends fully across the entrance to the air bleed channel 432 so as to obstruct the channel 432.

When the mouthpiece 409 is moved in the upstream longitudinal direction to activate the consumable 403, an elongate activation member (extending inwardly from the mouthpiece 409) engages the body 434 of the bung 433 and pushes the bung 433 in the upstream direction (see Figure 4B). This moves the enlarged head 435 of the bung 433 away from the entrance of the air bleed channel 432 such that the head 435 no longer obstructs the air bleed channel 432. This allows air to pass from the vapour flow passage 421 and into the storage chamber 416 (which, in turn, allows for flow of the first aerosol precursor from the storage chamber 416).

It should be noted that the bung sealing element shown in Figures 4A and 4B could be used in place of the pierceable membrane in Figures 3A and 3B and vice versa.

Additionally, unlike in the example described in Figures 3A to 3E, an external opening 436 (i.e. the opening outside the storage 416) of the air bleed channel 432 is located adjacent to the liquid transfer element 415. The liquid transfer element 415 defines a portion of the air bleed channel 432. The external opening 436 has a diameter of 0.5mm.

The external opening 436 and the liquid transfer element 415 are positioned such that a meniscus forms between them in use, the meniscus providing an obstruction which closes the air bleed channel 432. The air bleed channel 432 being closed prevents further liquid being transferred through the liquid transfer element 415 until the user draws on the device again, reducing leakage. Additionally, the obstruction may prevent leakage of liquid through the air bleed channel 432.

When the user draws on the device, the aerosol generator 422 forms an aerosol from aerosol precursor liquid, which causes the amount of aerosol precursor liquid in the liquid transfer element 415 to reduce.

The liquid transfer element 415 then absorbs further aerosol precursor liquid from the storage 416, which causes the liquid forming the obstruction to be pulled from the air bleed channel 432 and into the storage 416. This temporarily opens the air bleed channel 432 and permits air to enter the storage 416 to reduce the pressure difference between the storage and the external environment. Once the liquid transfer element 415 reaches a certain level of saturation with liquid, the obstruction forms again, closing the air bleed channel 432.

In other examples the external opening 436 is not directly adjacent to the liquid transfer element 415, but is sufficiently close for the obstruction to form.

The air bleed channel 432 follows a tortuous path. At the external opening 436 the air bleed channel 432 extends parallel to a longitudinal axis of the smoking substitute device in a longitudinal direction. The air bleed channel 432 turns away from the longitudinal direction towards a radially outward direction, before turning towards the longitudinal direction again.

The aerosol generating portion 422 of the liquid transfer element shown in the Figures 4A and 4B has a flattened upper (downstream) surface. Such a liquid transfer element could be used in the embodiment shown in Figure 3A and 3B.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined in the claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery device comprising:
a storage (416) for storing aerosol precursor liquid, the storage comprising an air bleed channel (432) for permitting air to enter the storage as the storage empties of aerosol precursor in use;
an aerosol generator for generating an aerosol from the aerosol precursor liquid for inhalation by a user; and
a liquid transfer element (415) for transferring aerosol precursor liquid from the storage to the aerosol generator,
**characterised in that** the air bleed channel and the liquid transfer element are configured such that aerosol precursor liquid from the liquid transfer element forms an obstruction in the air bleed channel in use to reduce flow through the air bleed channel,
wherein the aerosol delivery device is further configured such that the obstruction is removed to open the air bleed channel in response to a user drawing on the aerosol delivery device by causing the aerosol precursor liquid in the liquid transfer element to reduce and the obstruction to be pulled from the air bleed channel into the storage to open the air bleed channel.

2. An aerosol delivery device according to claim 1, the aerosol generator comprising an aerosol generator portion (422) configured to receive the aerosol precursor from the storage, the aerosol delivery device further comprising an air flow passage configured to direct air past the aerosol generator portion to pick up the aerosol precursor from the aerosol generator portion to form an aerosol.

3. An aerosol delivery device according to claim 2 and further comprising a member, the member comprising the liquid transfer element and the aerosol generator portion.

4. An aerosol delivery device according to any one of the preceding claims, wherein an external opening (436) of the air bleed channel has a diameter of less than 1 mm.

5. An aerosol delivery device according to claim 4, wherein the external opening has a diameter of substantially 0.5 mm.

6. An aerosol delivery device according to any one of the preceding claims, wherein an external opening of the air bleed channel is located adjacent to the liquid transfer element.

7. An aerosol delivery device according to claim 6, wherein the liquid transfer element defines a portion of the air bleed channel.

8. An aerosol delivery device according to any one of the preceding claims, wherein the air bleed channel follows a tortuous path.

9. An aerosol delivery device according to any one of the preceding claims, and further comprising a sealing element for inhibiting flow through the air bleed channel when in a deactivated state, wherein the sealing element is openable into an activated state to permit air flow through the air bleed channel when the obstruction is removed from the air bleed channel.

10. An aerosol delivery device according to claim 9, wherein the sealing element comprises a bung (433) received in the air bleed channel in the deactivated state, the bung movable into the activated state.

11. An aerosol delivery device according to claim 10, wherein the bung comprises an enlarged portion and a neck portion, wherein the enlarged portion extends fully across the air bleed channel to block the air bleed channel in the deactivated state, and the enlarged portion moves out of the air bleed channel and the neck portion moves into the air bleed channel when the bung is moved from the deactivated to the activated state, the neck portion extending partially across the air bleed channel.

12. An aerosol delivery device according to any one of the preceding claims, and further comprising a barrier arrangement for inhibiting flow of aerosol precursor from the storage to the liquid transfer element, wherein the barrier arrangement is openable so that the liquid transfer element can receive aerosol precursor from the storage.

13. An aerosol delivery device according to claim 12, wherein the liquid transfer element is movable to contact the barrier arrangement to open the barrier arrangement.

14. An aerosol delivery device according to any one of the preceding claims, wherein the aerosol delivery device is a consumable (403) for a vaping device.

15. An aerosol delivery device according to any one of the preceding claims and further comprising an additional aerosol generator, the additional aerosol generator configured to produce an additional aerosol from an additional aerosol precursor.

## Patentansprüche

1. Aerosolabgabevorrichtung, die Folgendes umfasst:
einen Speicher (416) zum Speichern einer Aerosolvorläuferflüssigkeit, wobei der Speicher einen Entlüftungskanal (432) umfasst, um Luft in den Speicher eindringen zu lassen, während der Aerosolvorläufer bei Verwendung aus dem Speicher austritt;
einen Aerosolgenerator zum Erzeugen eines Aerosols aus der Aerosolvorläuferflüssigkeit zur Inhalation durch einen Benutzer; und
ein Flüssigkeitstransportelement (415) zum Transport einer Aerosolvorläuferflüssigkeit aus dem Speicher zu dem Aerosolgenerator,
**dadurch gekennzeichnet, dass** der Entlüftungskanal und das Flüssigkeitstransportelement so ausgebildet sind, dass die Aerosolvorläuferflüssigkeit aus dem Flüssigkeitstransportelement bei Verwendung eine Obstruktion in dem Entlüftungskanal bildet, um die Strömung durch den Entlüftungskanal zu verringern,
wobei die Aerosolabgabevorrichtung außerdem derart ausgebildet ist, dass die Obstruktion entfernt wird, um den Entlüftungskanal zu öffnen, und zwar als Reaktion auf das Saugen eines Benutzers an der Aerosolabgabevorrichtung, wodurch die Aerosolvorläuferflüssigkeit in dem Flüssigkeitstransportelement verringert und die Obstruktion aus dem Entlüftungskanal in den Speicher gezogen wird, um den Entlüftungskanal zu öffnen.

2. Aerosolabgabevorrichtung nach Anspruch 1, wobei der Aerosolgenerator einen Aerosolgeneratorabschnitt (422) umfasst, der ausgebildet ist, um den Aerosolvorläufer aus dem Speicher aufzunehmen, wobei die Aerosolabgabevorrichtung außerdem einen Luftströmungsdurchgang umfasst, der ausgebildet ist, um Luft an dem Aerosolgeneratorabschnitt vorbeizuleiten, um den Aerosolvorläufer aus dem Aerosolgeneratorabschnitt aufzunehmen, um ein Aerosol zu bilden.

3. Aerosolabgabevorrichtung nach Anspruch 2, die außerdem ein Bauteil umfasst, wobei das Bauteil das Flüssigkeitstransportelement und den Aerosolgeneratorabschnitt umfasst.

4. Aerosolabgabevorrichtung nach einem der vorangegangenen Ansprüche, wobei eine äußere Öffnung (436) des Entlüftungskanals einen Durchmesser von weniger als 1 mm aufweist.

5. Aerosolabgabevorrichtung nach Anspruch 4, wobei die äußere Öffnung einen Durchmesser von im Wesentlichen 0,5 mm aufweist.

6. Aerosolabgabevorrichtung nach einem der vorangegangenen Ansprüche, wobei eine äußere Öffnung des Entlüftungskanals neben dem Flüssigkeitstransportelement angeordnet ist.

7. Aerosolabgabevorrichtung nach Anspruch 6, wobei das Flüssigkeitstransportelement einen Abschnitt des Entlüftungskanals definiert.

8. Aerosolabgabevorrichtung nach einem der vorangegangenen Ansprüche, wobei der Entlüftungskanal einem gewundenen Pfad folgt.

9. Aerosolabgabevorrichtung nach einem der vorangegangenen Ansprüche, die außerdem ein Abdichtungselement umfasst, um die Strömung durch den Entlüftungskanal in einem deaktivierten Zustand zu verhindern, wobei das Abdichtungselement in einen aktivierten Zustand öffenbar ist, um Luft durch den Entlüftungskanal strömen zu lassen, wenn die Obstruktion aus dem Entlüftungskanal entfernt ist.

10. Aerosolabgabevorrichtung nach Anspruch 9, wobei das Abdichtungselement einen Zapfen (433) umfasst, der in dem Entlüftungskanal in dem deaktivierten Zustand aufgenommen wird, wobei der Zapfen in den aktivierten Zustand bewegbar ist.

11. Aerosolabgabevorrichtung nach Anspruch 10, wobei der Zapfen einen vergrößerten Abschnitt und einen Halsabschnitt umfasst, wobei sich der vergrößerte Abschnitt vollständig über den Entlüftungskanal erstreckt, um den Entlüftungskanal in dem deaktivierten Zustand zu blockieren, und sich der vergrößerte Abschnitt aus dem Entlüftungskanal heraus und sich der Halsabschnitt in den Entlüftungskanal hinein bewegt, wenn der Zapfen von dem deaktivierten in den aktivierten Zustand bewegt wird, wobei sich der Halsabschnitt teilweise über den Entlüftungskanal erstreckt.

12. Aerosolabgabevorrichtung nach einem der vorangegangenen Ansprüche, die außerdem eine Absperranordnung umfasst, um die Strömung des Aerosolvorläufers von dem Speicher zu dem Flüssigkeitstransportelement zu verhindern, wobei die Absperranordnung öffenbar ist, sodass das Flüssigkeitstransportelement den Aerosolvorläufer aus dem Speicher aufnehmen kann.

13. Aerosolabgabevorrichtung nach Anspruch 12, wobei das Flüssigkeitstransportelement bewegbar ist, um die Absperranordnung zu berühren, um die Absperranordnung zu öffnen.

14. Aerosolabgabevorrichtung nach einem der vorangegangenen Ansprüche, wobei die Aerosolabgabevorrichtung ein Verbrauchsmaterial (403) für einen Verdampfer umfasst.

15. Aerosolabgabevorrichtung nach einem der vorangegangenen Ansprüche, die außerdem einen zusätzlichen Aerosolgenerator umfasst, wobei der zusätzliche Aerosolgenerator ausgelegt ist, um zusätzliches Aerosol aus einem zusätzlichen Aerosolvorläufer zu erzeugen.

## Revendications

1. Dispositif de distribution d'aérosol comprenant :
un dispositif de stockage (416) pour stocker un liquide précurseur d'aérosol, le dispositif de stockage comprenant un canal de purge d'air (432) pour permettre à de l'air d'entrer dans le dispositif de stockage lorsque le dispositif de stockage se vide de précurseur d'aérosol en utilisation ;
un générateur d'aérosol pour générer un aérosol à partir du liquide précurseur d'aérosol pour une inhalation par un utilisateur ; et
un élément de transfert de liquide (415) pour transférer un liquide précurseur d'aérosol du dispositif de stockage au générateur d'aérosol,
**caractérisé en ce que** le canal de purge d'air et l'élément de transfert de liquide sont configurés de telle sorte que du liquide précurseur d'aérosol provenant de l'élément de transfert de liquide forme une obstruction dans le canal de purge d'air en utilisation pour réduire un écoulement à travers le canal de purge d'air,
dans lequel le dispositif de distribution d'aérosol est en outre configuré de telle sorte que l'obstruction est retirée pour ouvrir le canal de purge d'air en réponse à un utilisateur aspirant sur le dispositif de distribution d'aérosol en amenant le liquide précurseur d'aérosol dans l'élément de transfert de liquide à diminuer et l'obstruction à être tirée à partir du canal de purge d'air dans le dispositif de stockage pour ouvrir le canal de purge d'air.

2. Dispositif de distribution d'aérosol selon la revendication 1, dans lequel le générateur d'aérosol comprend une partie de générateur d'aérosol (422) configurée pour recevoir le précurseur d'aérosol à partir du dispositif de stockage, le dispositif de distribution d'aérosol comprenant en outre un passage d'écoulement d'air configuré pour diriger de l'air au-delà de la partie de générateur d'aérosol afin de recueillir le précurseur d'aérosol à partir de la partie de générateur d'aérosol pour former un aérosol.

3. Dispositif de distribution d'aérosol selon la revendication 2 et comprenant en outre un élément, l'élément comprenant l'élément de transfert de liquide et la partie de générateur d'aérosol.

4. Dispositif de distribution d'aérosol selon l'une quelconque des revendications précédentes, dans lequel une ouverture externe (436) du canal de purge d'air présente un diamètre inférieur à 1 mm.

5. Dispositif de distribution d'aérosol selon la revendication 4, dans lequel l'ouverture externe présente un diamètre de sensiblement 0,5 mm.

6. Dispositif de distribution d'aérosol selon l'une quelconque des revendications précédentes, dans lequel une ouverture externe du canal de purge d'air est située adjacente à l'élément de transfert de liquide.

7. Dispositif de distribution d'aérosol selon la revendication 6, dans lequel l'élément de transfert de liquide définit une partie du canal de purge d'air.

8. Dispositif de distribution d'aérosol selon l'une quelconque des revendications précédentes, dans lequel le canal de purge d'air suit un trajet tortueux.

9. Dispositif de distribution d'aérosol selon l'une quelconque des revendications précédentes, et comprenant en outre un élément d'étanchéité pour empêcher un écoulement à travers le canal de purge d'air lorsqu'il est dans un état désactivé, dans lequel l'élément d'étanchéité peut être ouvert dans un état activé pour permettre un écoulement d'air à travers le canal de purge d'air lorsque l'obstruction est retirée du canal de purge d'air.

10. Dispositif de distribution d'aérosol selon la revendication 9, dans lequel l'élément d'étanchéité comprend une bonde (433) reçue dans le canal de purge d'air dans l'état désactivé, la bonde pouvant être déplacée dans l'état activé.

11. Dispositif de distribution d'aérosol selon la revendication 10, dans lequel la bonde comprend une partie élargie et une partie de col, dans lequel la partie élargie s'étend complètement à travers le canal de purge d'air pour bloquer le canal de purge d'air dans l'état désactivé, et la partie élargie se déplace hors du canal de purge d'air et la partie de col se déplace dans le canal de purge d'air lorsque la bonde est déplacée de l'état désactivé à l'état activé, la partie de col s'étendant partiellement à travers le canal de purge d'air.

12. Dispositif de distribution d'aérosol selon l'une quelconque des revendications précédentes, et comprenant en outre un agencement de barrière pour inhiber un écoulement de précurseur d'aérosol à partir du dispositif de stockage vers l'élément de transfert de liquide, dans lequel l'agencement de barrière peut être ouvert de sorte que l'élément de transfert de liquide peut recevoir un précurseur d'aérosol à partir du dispositif de stockage.

13. Dispositif de distribution d'aérosol selon la revendication 12, dans lequel l'élément de transfert de liquide est mobile pour venir en contact avec l'agencement de barrière afin d'ouvrir l'agencement de barrière.

14. Dispositif de distribution d'aérosol selon l'une quelconque des revendications précédentes, dans lequel le dispositif de distribution d'aérosol est un produit consommable (403) pour un dispositif de vapotage.

15. Dispositif de distribution d'aérosol selon l'une quelconque des revendications précédentes et comprenant en outre un générateur d'aérosol supplémentaire, le générateur d'aérosol supplémentaire étant configuré pour produire un aérosol supplémentaire à partir d'un précurseur d'aérosol supplémentaire.
